(19) Europäisches Patentamt
European Patent Office
Office européen des brevets

(11) EP 4 549 616 A1

(12) EUROPEAN PATENT APPLICATION

(43) Date of publication:
**07.05.2025 Bulletin 2025/19**

(21) Application number: **24207972.1**

(22) Date of filing: **22.10.2024**

(51) International Patent Classification (IPC):
***C25B 1/04** (2021.01)* ***C25B 15/08** (2006.01)*
***C01B 4/00** (2006.01)* ***C01C 1/04** (2006.01)*
***C07B 59/00** (2006.01)* ***C07C 209/26** (2006.01)*

(52) Cooperative Patent Classification (CPC):
**C25B 1/04; C01B 4/00; C01C 1/0405; C07B 59/001; C07C 209/26; C25B 15/081**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 EP 23206996**

(71) Applicant: **BASF SE**
**67056 Ludwigshafen am Rhein (DE)**

(72) Inventors:
- **Vossen, Marcus**
**67117 Limburgerhof (DE)**
- **Ernst, Martin**
**67056 Ludwigshafen am Rhein (DE)**
- **Stock, Christoph**
**67056 Ludwigshafen am Rhein (DE)**
- **Harthausen, Sina**
**67056 Ludwigshafen am Rhein (DE)**
- **Xiong, Jiawen**
**67056 Ludwigshafen am Rhein (DE)**
- **Hueffer, Stephan**
**67063 Ludwigshafen (DE)**
- **Krueger, Marco**
**67056 Ludwigshafen am Rhein (DE)**
- **Weiss, Thomas**
**67056 Ludwigshafen am Rhein (DE)**

(74) Representative: **BASF IP Association**
**BASF SE**
**GBI - Z078**
**67056 Ludwigshafen (DE)**

# (54) PROCESS FOR MAKING AMINES FROM CARBONYL COMPOUNDS USING HYDROGEN HAVING LOW DEUTERIUM CONTENT PRODUCED WITH NON-FOSSIL ENERGY

(57) A process for the preparation of amines comprising the following steps:
(a) providing hydrogen with a molar share of deuterium ≤ 100 ppm, based on the total hydrogen content, by electrolysis of water using electrical power generated at least in part from non-fossil energy,
(b) reacting the hydrogen from step (a) with nitrogen to form ammonia,
(c) reacting the ammonia from step (b) with a carbonyl compound (I)

$R_1R_2C{=}O$ (I)

in the presence of hydrogen from step (a) to form the corresponding amine (II)

$R_1R_2HC\text{-}NH_2$ (II).

EP 4 549 616 A1

## Description

The present invention relates to a process for making amines having a lower deuterium content, based on the total hydrogen content, compared to amines generated by reaction with hydrogen produced from petrochemical processes using natural gas, condensate or petroleum oil, the amines obtained thereby as well as to their use. More specifically, the present invention relates to a process for making amines using hydrogen having low deuterium content produced from non-fossil electrical energy, the amines obtained thereby as well as to the use of the molar share of deuterium in the hydrogen bound in the amines for tracing the origin of the hydrogen.

In the preparation of amines from carbonyl compounds, more particularly aldehydes and ketones, ammonia is a key precursor. Since the development of the Haber-Bosch process for the preparation of ammonia, the vast majority of ammonia is manufactured by the direct synthesis from hydrogen and nitrogen in the presence of a catalyst, especially an iron-containing catalyst. Special care needs to be taken with the provision of the starting materials hydrogen and nitrogen. They should exhibit a high purity and be substantially free from catalyst poisoning agents such as carbon monoxide and sulfur compounds such as $H_2S$ and $SO_2$. In modern processes, a significant amount of the hydrogen is provided by steam reforming, thus, from natural gas.

However, the petrochemical steam reforming process has its negative impacts with regard to its carbon footprint including the consumption of a lot of fossil-based natural resources and energy.

It is therefore an object of the present invention to provide environmentally friendly amines in an environmentally friendly process for making the same, that process using as little fossil-based energy as possible.

The object is achieved by a process for the preparation of amines, wherein said process comprises the following steps:

(a) providing hydrogen with a molar share of deuterium ≤ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content, by electrolysis of water using electrical power generated at least in part from non-fossil energy,
(b) reacting the hydrogen from step (a) with nitrogen to form ammonia,
(c) reacting the ammonia from step (b) with a carbonyl compound (I)

$$R_1R_2C{=}O \qquad (I)$$

in the presence of hydrogen from step (a) to form the corresponding amine (II)

$$R_1R_2HC\text{-}NH_2 \qquad (II).$$

In a further embodiment of the present invention, the object is achieved by amines wherein the molar share of deuterium in the $R_1R_2HC\text{-}NH_2$ group or groups formed in step (c) is ≤ 100 ppm, preferably in the range of from 10 to 95 ppm, more preferably in the range of from 15 to 90 ppm, most preferably in the range of from 20 to 80 ppm, especially in the range of from 30 to 75 ppm based on the total hydrogen content of said $R_1R_2HC\text{-}NH_2$ group or groups.

It is important that the origin of the hydrogen and downstream compounds obtained by clean energy can be tracked in a reliable way. This is especially important to ensure that:

- Hydrogen and downstream compounds have been produced in accordance with sustainability criteria.
- Renewable attributes aren't subject to double counting.

Companies are placing increasing importance on sourcing green energy. Because of this, tracking systems have to be developed for the origin of the energy used in the preparation of hydrogen and downstream compounds.

This object is also achieved by use of the molar share of deuterium in hydrogen bound in downstream compounds based on hydrogen for tracing the origin, especially the energetic origin, of the hydrogen bound in the downstream compounds based on hydrogen, wherein the compounds are amines and a process for tracing the origin, especially the energetic origin, of hydrogen bound in downstream compounds based on hydrogen by determining the molar share of deuterium in the hydrogen bound in said downstream compounds. Methods for determination of the molar share of deuterium in hydrogen and in downstream compounds based on hydrogen are known to a person skilled in the art and include mass spectrometry and NMR technologies.

A further environmental benefit of the environmentally friendly amines according to the present invention is their use in carbon capturing processes, since the amines according to the present invention are produced using as little fossil-based energy as possible, ideally no fossil-based energy, and do therefore only add as little as possible, ideally nothing, to

$CO_2$ emission.

A further embodiment of the present invention is therefore the use of the amines according to the present invention as liquid or solid $CO_2$ absorbents in $CO_2$ capturing processes. Specific examples of amines usable with preference are:

i) 2-aminoethoxyethanol (ADEG), 2-aminoethanol (monoethanolamine), 2-(methylamino)ethanol, 2-(ethylamino) ethanol, 2-(n-butylamino)ethanol, 2-amino-2-methylpropanol, N-(2-aminoethyl)piperazine, methyldiethanolamine, ethyldiethanolamine, dimethylaminopropanol, tert-butylaminoethoxyethanol (TBAEE), methoxyethoxyethyl-tert-butylamine, 2-amino-2-methylpropanol, diisopropanolamine (DIPA or DIPOA);

ii) 3-methylaminopropylamine, ethylenediamine, diethylenetriamine, triethylenetetramine, 2,2-dimethyl-1,3-diaminopropane, hexamethylenediamine, 1,4-diaminobutane, 3,3-iminobispropylamine, tris(2-aminoethyl)amine, bis(3-dimethylaminopropyl)amine and tetramethylhexamethylenediamine;

iii) piperazine, 2-methylpiperazine, N-methylpiperazine, 1-hydroxyethylpiperazine, 1,4-bishydroxyethylpiperazine, 4-hydroxyethylpiperidine, homopiperazine, piperidine, 2-hydroxyethylpiperidine, triethylendiamine (TEDA) and morpholine; and

iv) mixtures thereof.

The molar share of deuterium in hydrogen and downstream compounds based on hydrogen is given in the present application in ppm, based on the total hydrogen content, which is the mol-ppm content of deuterium, based on the total hydrogen content (in hydrogen or in the compounds discussed, respectively).

The deuterium content of hydrogen and downstream compounds based on hydrogen is given in the present application in atom-ppm based on the total molar hydrogen content (total atoms of protium $^1H$ and deuterium $^2H$). The terms "deuterium content" and "molar share of deuterium" are used synonymously throughout the application.

In physical organic chemistry, a kinetic isotope effect is the change in the reaction rate of a chemical reaction when one of the atoms in the reactants is replaced by one of its isotopes. Formally, it is the ratio of rate constants $k_L$ / $k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently a slower reaction rate.

Isotopic rate changes are most pronounced when the relative mass change is greatest, since the effect is related to vibrational frequencies of the affected bonds. For instance, changing a hydrogen atom (H) to its isotope deuterium (D) represents a 100 % increase in mass, whereas in replacing $^{12}C$ with $^{13}C$, the mass increases by only 8 percent. The rate of a reaction involving a C-H bond is typically 6-10 times faster than the corresponding C-D bond, whereas a $^{12}C$ reaction is only 4 percent faster than the corresponding $^{13}C$ reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Step (a)

Step (a) concerns the electrolysis of water using electrical power generated at least in part from non-fossil energy.

Electrolysis of water is an environmentally friendly method for production of hydrogen because it uses renewable $H_2O$ and produces only pure oxygen as by-product. Additionally, water electrolysis utilizes direct current (DC) from sustainable energy resources, for example solar, wind, hydropower and biomass.

It is observed that by electrolysis of water, the deuterium atom content of the hydrogen is lower than in the hydrogen generated petrochemically, for example as contained in synthesis gas, in general $\leq$ 100 ppm, preferably in general $\leq$ 90 ppm, for example from 30 to 75 ppm. The deuterium atom content in electrolytically produced hydrogen may be as low as 10 ppm. The deuterium is mainly present in the form of D-H rather than $D_2$.

One suitable water electrolysis process is alkaline water electrolysis. Hydrogen production by alkaline water electrolysis is a well-established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions (OH-). The produced $H_2$ emanates from the cathode surface in gaseous form and the hydroxyl ions (OH-) migrate under the influence of the electrical field between anode and cathode through the porous diaphragm to the anode, where they are discharged to half a molecule of oxygen ($O_2$) and one molecule of water ($H_2O$). Alkaline

electrolysis operates at lower temperatures such as 30-80°C with alkaline aqueous solution (KOH/NaOH) as the electrolyte, the concentration of the electrolyte being about 20% to 30 %. The diaphragm in the middle of the electrolysis cell separates the cathode and anode and also separates the produced gases from their respective electrodes, avoiding the mixing of the produced gases. However, alkaline electrolysis has negative aspects such as limited current densities (below 400 $mA/cm^2$), low operating pressure and low energy efficiency.

In one preferred embodiment of the inventive process, hydrogen is provided by polymer electrolyte membrane water electrolysis. Variants of polymer electrolyte membrane water electrolysis are proton exchange membrane water electrolysis (PEMWE) and anion exchange membrane water electrolysis (AEMWE).

PEM water electrolysis was developed to overcome the drawbacks of alkaline water electrolysis. PEM water electrolysis technology is similar to the PEM fuel cell technology, where solid polysulfonated membranes (Nafion®, fumapem®) are used as an electrolyte (proton conductor). These proton exchange membranes have many advantages such as low gas permeability, high proton conductivity ($0.1 \pm 0.02$ S $cm^{-1}$), low thickness (20-300 $\mu$m), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A $cm^{-2}$), high efficiency, fast response, operation at low temperatures (20-80°C) and production of ultrapure hydrogen. The state-of-the-art electrocatalysts for PEM water electrolysis are highly active noble metals such as Pt/Pd for the hydrogen evolution reaction (HER) at the cathode and $IrO_2$/$RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind and solar power, where sudden spikes in energy output would otherwise result in uncaptured energy. The polymer electrolyte allows the PEM water electrolyzer to operate with a very thin membrane (ca. 100-200 $\mu$m) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA, or Nafion®, a DuPont product. While Nafion® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

An overview over hydrogen production by anion exchange membrane water electrolysis is given in H. A. Miller et al., Sustainable Energy Fuels, 2020, 4, pp. 2114 - 2133.

K. Harada et al., International Journal of Hydrogen Energy 45 (2020), pp. 31389 - 31 395 report a deuterium depletion by a factor from 2 to 3 in polymer electrolyte membrane water electrolysis. The separation factor $\beta$

$$\beta = ([H]/[D])_{gas} / ([H]/[D])_{liquid}$$

where "gas" is the evolved gas and "liquid" is water before the electrolysis was found to be between 2 and 3 at current densities of from 1.0 to 2.0 A $cm^{-2}$, corresponding to a stoichiometric number A of between 4 and 9 at the given water mass flow in the anode. The stoichiometric number A is defined as follows:

$$\lambda = V \times \rho / (J/2F \times 60 \times M_{H2O})$$

where V (mL $min^{-1}$) is the water mass flow in the anode, F is the Faraday constant, J is electrolysis current (A), $\rho$ is the density of water (g $mL^{-1}$) and $M_{H2O}$ (g $mol^{-1}$) is the molar weight of water. A stoichiometric number A of 10 means that 10 times the amount of fresh water than can be theoretically consumed by electrolysis at the given electrolysis current is

supplied to the anode.

H. Sato et al., International Journal of Hydrogen Energy 46 (2021), pp. 33 689 - 33 695, report for anion exchange membrane water electrolysis that deuterium concentration in the evolving hydrogen gas is diluted by approximately 1/5 against the feed water, at A = 4.

Hence, deuterium in the evolving hydrogen gas can easily be depleted by a factor of from 2 to 5 with regard to feed water in polymer electrolyte membrane water electrolysis. Depending on the electrolysis conditions (water flow, current density), even higher depletion factors are possible. Since the average deuterium content of water is about 150 ppm, based on the total hydrogen content, hydrogen provided in step (a) of the inventive process may have a deuterium content of from 30 to 75 ppm, based on the total hydrogen content, or even lower.

The electrical power is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably $\leq$ 50%, preferably $\leq$ 30%, most preferably $\leq$ 20%.

The electrical power from non-fossil resources used in water electrolysis according to the invention can be generated by nuclear energy. Nuclear energy is considered renewable by the European Commission, as long as certain preconditions (inter alia safe long-term storage of nuclear waste) are fulfilled.

The electrical power from non-fossil resources used in water electrolysis according to the invention is preferably generated from wind power, solar energy, biomass, hydropower and geothermal energy.

In one preferred embodiment of the inventive process, the electrical power used in water electrolysis is generated from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the electrical power used in electrolysis is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants; dry steam, flash and binary. Dry steam is the oldest form of geothermal technology and takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

In one further preferred embodiment of the inventive process, the electrical power used in water electrolysis is generated from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in water electrolysis is generated from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling has by far the highest efficiency among all solar energy technologies.

In one further preferred embodiment of the inventive process, the electrical power used in water electrolysis is generated from biomass. Biomass is biological material derived from living, or recently living organisms. It most often refers to plants or plant-derived materials which are specifically called lignocellulosic biomass. As an energy source, biomass can either be used directly via combustion to produce heat or electricity, or indirectly after converting it to various forms of biofuel. Conversion of biomass to biofuel can be achieved by different methods which are broadly classified into: thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare

with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Step (b)

Step (b) concerns the reaction of the hydrogen from step (a) with nitrogen to form ammonia.

The reaction of step (b) preferably follows the Haber-Bosch process.

The catalysts usually used in the Haber-Bosch process generally fall into one of two categories, fused-iron and supported metallic catalysts. Fused-iron catalysts are derived from iron oxides, of which there are three possibilities: $Fe_2O_3$, $Fe_3O_4$, and $Fe_{1-x}O$, which are known as hematite, magnetite, and wüstite, respectively. Industrially, these iron catalysts will be multipromoted with promoters such as $K_2O$, BaO, KOH, CaO, MgO and $Al_2O_3$ are present in small quantities of a few weight percent. Supported metallic catalysts are catalysts made up of a metallic catalyst material, normally ruthenium or cobalt for the ammonia synthesis reaction, present on the surface of a support material, normally activated carbon or a metal oxide. Commonly the weight percentage of the metallic catalyst is around 2-10%.

Other catalysts which may be used are nickel, and nitride catalyst systems or electride, hydride, nitride, oxynitride hydride promoted Ru, Fe, Co, and Ni catalysts.

The catalysts mentioned above can be used for both conventional centralized large-scale Haber-Bosch ammonia synthesis plants and distributed small-scale ammonia production via the same process.

In one embodiment of the present invention, step (b) is performed at a pressure in the range of from 50 to 350 bar (abs), preferably 150 to 300 bar (abs).

In one embodiment of the present invention, step (b) is performed at a temperature in the range of from 300 to 600 °C, preferably 400 to 500 °C.

By performing step (b), ammonia is formed. The deuterium content of the ammonia is significantly lower than obtained by classical petrochemical routes. The deuterium content is in general $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content.

The overall kinetic isotope effect is cumulative, since it will also be present in all subsequent production steps downstream the value chain.

Step (c)

In step (c), the ammonia from step (b) is reacted with a carbonyl compound (I)

$$R_1R_2C{=}O \qquad \text{(I)}$$

in the presence of hydrogen from step (a) to form the corresponding amine (II)

$$R_1R_2HC\text{-}NH_2 \qquad \text{(II).}$$

In general, $R_1$ and $R_2$ are aliphatic, cycloaliphatic or aromatic residues comprising 1 to 20 carbon atoms.

An overview of the process of reductively aminating carbonyl compounds in the presence of ammonia has been given in Roose, P., Eller, K., Henkes, E., Rossbacher, R. and Hoke, H. (2015). Amines, Aliphatic, in Ullmann's Encyclopedia of Industrial Chemistry, https://doi.org/10.1002/14356007.a02_001.pub2 and Roose, P. and Turcotte, M.G. (2016); Amines, lower aliphatic, in Kirk-Othmer Encyclopedia of Chemical Technology, John Wiley & Sons, Inc (Ed.), https://doi.org/10.1002/0471238961.1215230520211803.a01.pub3.

The reductive amination actually comprises two separate chemical steps. In step one, a ketone or aldehyde is converted with ammonia into an imine. In step two, the imine is hydrogenated. Both reactions require different kinds of catalysts; the first step requires an acidic catalyst because imine formation is accelerated by acidic additives as described in patent EP 0 713 861 A1 (BASF AG) or in the review by R. Patil and S. Adimurthy in Asian J. Org. Chem. 2013, 2, 726 - 744. The second step requires a hydrogenation catalyst. The process can be catalyzed with a heterogeneous or homogenous catalyst.

In the case of heterogeneous catalysis, these steps are sometimes effectively separated, e.g., in the case of IPDA there are numerous descriptions of a process in which the isophoronenitrile is first fed into a reactor filled with a weakly acidic or acidic catalyst like aluminum oxide or titanium oxide (EP 3 050 870 A1, Evonik AG, EP 0 449 089 A1, BASF AG). Then, the crude iminonitrile enters a hydrogenation reactor in which the imine and the nitrile groups are hydrogenated, optionally at a different temperature and hydrogen pressure. By combining an acidic support with a metal that serves as hydrogenation catalyst, both steps can be carried out in one pot without separate reaction zones or spaces. The carrier material of any hydrogenation catalyst could also serve as an imination catalyst, e.g., aluminum oxide or zirconium oxide. The acidic catalyst can also simply be added to the hydrogenation catalyst when the process is operated in a batch, fed-batch or continuous stirred tank reactor system. Thus, powdered metal oxides can be added to a stirred tank reactor in combination with a slurry hydrogenation catalyst such as Raney-Nickel or Raney-Co or Pd/C. In such cases it might be advantageous to ramp up the temperature or the hydrogen supply in order to allow the imination to proceed before the hydrogenation occurs in order to prevent undesired hydrogenation of the carbonyl compound to an alcohol.

Generally, the catalyst is composed of various active metals on an oxidic support, and either extruded or shaped into pellets or other more intricate shaped bodies. The active metals are selected from transition metals, preferably from Ni, Co, Cu, Ag, Au, Pd, Pt, Rh, Ru, Ir, Fe. Optionally, other metals can be added to moderate the activity and selectivity of the catalytically active metals, like Mo, Sb, Bi, Sn, Mn. Other elements like rare earth metals, e.g., La or Y, and main group elements like Li, Na, K, Ca, Ba, Cs, P, S, or carbon may also be present and can enhance mechanical stability, selectivity or lifetime of the catalysts. Preferred are Ni, Co, Cu, Ag, Pd, Pt, Ru as active metals, and Sn, Cr, Mo, Mn, La, Li, Na, P as further admixtures. Supports are chosen from aluminum oxide, silicon oxide, zirconium oxide, titanium oxide, active carbon, preferably from aluminum oxide, silicon oxide, zirconium oxide or mixtures of these metal oxides. Optionally, particles of elemental metals and binders like cement can also be added to the catalyst to facilitate the shaping or prolonging the lifetime. Suitable catalysts are also so-called Raney-type catalysts which are made by leaching a metal-aluminum alloy (typically Ni or Co-Al alloy) with NaOH. The resulting macroporous catalysts are highly active and can be used as wet slurries in suspension catalysis or shaped with appropriate binders into fixed bed catalysts. Such catalysts can also be produced in a foam-like structure or hollow spheres depending on whether the alloy is applied to an appropriate support before leaching. The support may be removed at any stage of this process. The Raney-type catalysts can be promoted by various metals in minor amounts, e.g., Cr, Fe, Mo and the like. As described above, in the case of suspension catalysts like Raney-type catalysts or noble metal catalysts with active carbon or metal oxides as support, it is advantageous to add an acidic powdered compound that catalyzes the imination step, e.g., $TiO_2$ or $ZrO_2$.

For the synthesis in the gas phase, the reactants are fed to the reactor in a controlled manner, preferably in a cycle gas stream, evaporated and in gaseous form. Suitable aldehydes and ketones for a gas phase synthesis are those which, owing to their boiling points and the boiling points of their reaction products, can be kept in the gas phase within the process parameters by process technology means. Examples are acetaldehyde, propanal, acetone, isbutyraldehyde, butanal, cyclohexanone, cyclopentanone. For isopropylamine, the process is described in US 2001/0003137 A1. The cycle gas serves firstly to evaporate the reactants and secondly as a reactant for the amination.

In the cycle gas method, the starting materials (aldehyde or ketone, hydrogen and ammonia) are evaporated in a cycle gas stream and fed to the reactor in gaseous form. The reactants (aldehyde or ketone, hydrogen and ammonia) may also be evaporated as aqueous solutions and passed to the catalyst bed with the cycle gas stream. Advantageously, the carbonyl compound and the ammonia stream are evaporated separately and are mixed in close proximity of the reaction zone. Preferred reactors are tubular reactors. Examples of suitable reactors with cycle gas stream can be found in Ullmann's Encyclopedia of Industrial Chemistry, 5th Ed., Vol. B 4, pages 199-238, "Fixed-Bed Reactors". Alternatively, the reaction is advantageously carried out in a tube bundle reactor or in a single-stream plant. In a single-stream plant, the tubular reactor in which the reaction proceeds can consist of a series connection of a plurality of (e.g., two or three) individual tubular reactors. Optionally, an intermediate introduction of feed (comprising the reactant and/or ammonia and/or $H_2$) and/or cycle gas and/or reactor effluent from a downstream reactor is possible here in an advantageous manner.

The cycle gas flow rate is preferably in the range from 40 to 1500 $m^3$ (at operating pressure)/[$m^3$ of catalyst (bed volume) x h], in particular in the range from 100 to 700 $m^3$ (at operating pressure)/[$m^3$ of catalyst (bed volume) x h]. The cycle gas comprises preferably at least 10% by volume, particularly from 20 to 100% by volume, very particularly from 50 to 100% by volume of $H_2$. Flow through the fixed bed of catalyst can be either from the top or from the bottom. The temperature, pressure and amount of the gas stream are set so that even relatively high-boiling reaction products remain in the gas phase. Ammonia (nitrogen compound) is preferably used in a from 0.90- to 100-fold molar amount, in particular in a from 1.0- to 10-fold molar amount, in each case based on the carbonyl compound used. As primary amines react faster than ammonia with the starting aldehyde or ketone, secondary and tertiary amines can be formed consecutively. The proportion of the various amines can be controlled by varying the excess of ammonia. Similarly, whenever there is a possibility for cyclizing, e.g., when several carbonyl groups are present in proximity to each other, the tendency to form a cycle can be controlled by varying the excess of ammonia. A greater excess will lead to more of the open-chain product. The process of the invention is preferably carried out at an absolute pressure in the range from 1 to 300 bar, preferably from 1 to 50 bar, particularly preferably from 1 to 30 bar.

The process of the invention is preferably carried out at a temperature in the range from 80 to 300°C, preferably from 150 to 250°C, particularly preferably from 170 to 230°C. The process is preferably operated with an amount of off-gas of from 5 to 800 standard cubic meters/h, in particular from 20 to 300 standard cubic meters/h. [Standard cubic meters

(standard $m^3$)=volume converted to STP]. The space velocity over the catalyst is preferably in the range from 0.1 to 3.0 kg, preferably from 0.1 to 2.0 kg, particularly preferably from 0.2 to 1.5 kg, of carbonyl compound per liter of catalyst (bed volume) and hour. The use of higher temperatures, higher total pressures and higher space velocities over the catalyst is possible. The pressure in the reactor, which is the sum of the partial pressures of the aminating agent, the carbonyl component and the reaction products formed at the temperatures indicated, is advantageously increased to the desired reaction pressure by injection of hydrogen. The water of reaction formed during the reaction generally does not have any adverse effect on the conversion, the reaction rate, the selectivity and the operating life of the catalyst and is therefore advantageously removed from the reaction product only in the work-up of the reaction product, e.g., by distillation. The reaction product mixture is advantageously depressurized and the excess hydrogen and any excess aminating agent present are then removed and the crude reaction product obtained is purified, e.g. by means of fractional rectification. Suitable work-up methods are described, for example, in EP 1 312 600 A and EP 1 312 599 A (both BASF AG). Unreacted starting materials and any suitable byproducts obtained can be recirculated to the synthesis. After condensation of the products in a separator, unreacted starting materials can once again be passed, in discontinuous or continuous operation, in the circulating gas stream over the catalyst bed.

In the liquid phase, the process can be carried out in continuous mode or in batch- or fed-batch mode. Preference is given to continuous or semi-continuous (fed-batch) mode. Reactors can be for example tubular reactors, multi-tube reactors, shaft reactors, shell-tube reactors, continuous stirred tank reactors, cascades of such reactors, jet-loop reactors, bubble columns, gas lift reactors, stirred tank reactors or combinations thereof. The process is preferably carried out in continuous mode, and the catalyst is preferably arranged as a fixed bed in the reactor. Flow through the fixed bed of catalyst can be either from the top or from the bottom. In addition to the liquid phase, a gas phase may be present. The reactants (aldehyde or ketone, plus ammonia) are passed simultaneously, including hydrogen, over the catalyst at pressures of generally 5 to 50 MPa (50-500 bar), preferably 7 to 35 MPa, more preferably 10 to 19 MPa, and temperatures of generally 30 to 350°C, particularly 40 to 300°C, preferably 50 to 280°C, more preferably 60 to 250°C, in particular 70 to 240°C. The catalyst hourly space velocity is generally in the range from 0.05 to 5 kg, preferably 0.1 to 2 kg and more preferably 0.2 to 1.5 kg of aldehyde or ketone per liter of catalyst (bed volume) and hour. The molar ratio of hydrogen to aldehyde or ketone used is generally from 1:1 to 40:1, preferably from 2:1 to 10:1. The hydrogen can be returned as recycle gas to the reaction.

Ammonia is generally added in molar ratios to the carbonyl group of from 0.5:1 to 500:1, preferably from 2:1 to 40:1. The reaction can also be carried out in the presence of water. However, the water content should be not more than 10% by weight, preferably less than 5% by weight, particularly preferably less than 3% by weight. If appropriate, the reactants can be diluted with a suitable solvent such as tetrahydrofuran, dioxane, N-methylpyrrolidone or ethylene glycol dimethyl ether. It is appropriate to heat the reactants before they are fed into the reaction vessel, preferably to the reaction temperature. It is possible to use higher temperatures and higher overall pressures and catalyst hourly space velocities. The pressure in the reaction vessel, which results from the sum of the partial pressures of ammonia, aldehyde or ketone, and of the reaction products formed and, if appropriate, of the solvent used at the temperatures specified, is appropriately increased by injecting hydrogen up to the desired reaction pressure. The excess ammonia can be circulated together with the hydrogen. In order to remove the heat of reaction from the catalyst bed, it is advantageous to recirculate part of the crude product obtained in the gas-liquid separator which may still contain some ammonia. It is advantageous to work with high cross-sectional loadings of reactants, i.e., to maintain a suitably high amount of reactant flow in relation to the diameter of the tubular reactor. Suitable cross-sectional loadings are in the range of 5-50 $kg/m^2/s$ as described in US 2012/0245390 A1 (BASF SE). Such cross-sectional loadings ensure sufficient mass transfer from the gas phase (hydrogen) into the liquid phase and to the catalyst surface. In the case of slurry processes, this mass transfer is brought about by sufficiently high stirring speed, mixing with a pump or in a jet. After the reaction effluent has been decompressed appropriately, the excess hydrogen and ammonia present are removed and the resulting crude reaction product is purified, for example by a fractional rectification. The excess ammonia and the hydrogen are advantageously returned back into the reaction zone. The same applies to any incompletely converted carbonyl compounds.

The homogeneously catalyzed reductive amination can in principle be carried out in the liquid phase much like the heterogeneously catalyzed reductive amination. However, the catalyst(s) are neither slurry nor fixed-bed catalysts but metal complexes, Lewis acids, and the like. In some cases, the ketones used as starting materials are prochiral, i.e., depending on the direction of attack of a nucleophile, from above or from below the plane of the C=O group, chiral compounds with opposite chirality are obtained. This is the case when both substituents of a ketone are not the same. In the case of homogeneous catalysis, when chiral ligands are employed, this can lead to a differentiation of the direction of addition of hydrogen resulting in products which are enriched in one enantiomer. If achiral ligands are employed, racemic products are obtained, as with heterogeneous catalysts. Such an enantioselective process is described in WO 2016/189129 A1 (BASF SE). In similar processes, ammonia is not used directly as the aminating agent but in the form of ammonium salts or formamide. In a publication in Chem. Eur. J. 2014, 20, 245-252 the reductive amination is carried out with ammonium formate as ammonia and hydrogen donor. In patent US2004/0267051, ammonium formate is also used as reductant, but ammonia is added in excess as a solution in MeOH. These processes are still in accordance with the

invention since ammonia with a low share of deuterium can be employed in the preceding step in which the ammonia donor is synthesized by addition of an acid or derivatization with other reagents. Similarly, the hydrogen with a low share of deuterium can be used to produce formic acid with a low share of deuterium which is then combined with the ammonia to give ammonium formate with a low share of deuterium.

The invention also pertains to other forms of ammonia or hydrogen that may be employed as substitutes for pure ammonia and hydrogen. This includes transfer hydrogenations in which an amine or alcohol is dehydrogenated and transfers its hydrogen atoms to another carbonyl compound. In this case, the hydrogenation donor can be manufactured with hydrogen with a low deuterium share which makes the transfer agent a source of hydrogen with a low deuterium share.

Primary amines are compounds which have at least one primary amino group ($-NH_2$). Primary diamines have two primary amino groups. Primary triamines have three primary amino groups. Primary polyamines have more than three primary amino groups. Primary amines are valuable products with a large number of different uses, for example as solvents, stabilizers, for the synthesis of chelating agents, as starting materials for producing synthetic resins, inhibitors, interface-active substances, intermediates in the manufacture of fuel additives (U.S. Pat. No. 3,275,554 A, DE 2125039A and DE36 11 230 A), surfactants, drugs and crop protection agents, hardeners for epoxy resins, catalysts for polyurethanes, intermediates for producing quaternary ammonium compounds, plasticizers, corrosion inhibitors, synthetic resins, ion exchangers, textile auxiliaries, dyes, vulcanization accelerators and/or emulsifiers. Primary diamines and triamines are valuable products with a large number of different uses, for example as solvents, stabilizers, for the synthesis of chelating agents, as starting materials for producing synthetic resins, drugs, inhibitors, corrosion protectants, polyurethanes, as hardeners for epoxy resins, and interface-active substances.

Preferred amines that can be prepared according to the invention are

a) Alkylamines and aliphatic diamines, such as ethylamine, diethylamine, triethylamine, propylamine, dipropylamine, 3-dimethylamino-1-propylamine, butylamine, isobutylamine, dibutylamine, diisobutylamine, 2-ethylhexylamine, bis(2-ethylhexyl)amine, octylamine, 2-propylheptylamine, isononylamine, 3,5,5-trimethylhexylamine, tridecylamine, ditridecylamine, diethylaminoethylamine, diisopropylaminoethylamine, isopropylamine, diisopropylamine, adamantylamine, cyclopentylamine, cyclohexylamine, menthylamine, aminated glucose, piperidine, triacetonediamine, isophoronediamine and aminomethylpinane;
b) Alkanolamines, such as 2-aminobutanol, 3-aminobutanol, 2-aminopropanol and 3-aminopropanol;
c) Aromatic amines, such as benzylamine, 1-phenylethylamine, aminoindane, dimethylaminoindane, benzylamine, 4-methyl-2-phenylethylamine, 2,6-dimethylaniline, orthophthalodiamine and meta-xylylene diamine;
d) Biobased amines, such as 2-octylamine, furfurylamine, tetrahydrofurfurylamine and isosorbide diamine, isomannide diamine, aminated sorbitol, bisaminomethylfurane and bisaminomethyltetrahydrofurane.

The plural denotes all congeners, i.e., the mono, di and trialkylated amine of the respective hydrocarbon substituent. The preferred amines can be used as curing agents for epoxy composite materials, as polyurethane catalysts or building blocks for such catalysts, as neutralizing agents, feedstocks for detergents, auxiliaries and solvents in various kinds of production, e.g., the lyocell process, as building blocks for agrochemical and pharmaceutical active ingredients and the like.

These amines can be prepared according to the processes described above from aldehydes and ketones. Amines are obtained from (i) aliphatic aldehydes, such as acetaldehyde, propionic aldehyde, butyraldehyde, isobutyraldehyde, hexanal, 2-ethylhexanal, 2-propylheptanal, isotridecanal, 3,5,5-trimethylhexanal, formylpinane, or dialdehydes like glutaraldehyde (giving rise to piperidine); (ii) aliphatic ketones, such as acetone, isophoronenitrile, adamantone, menthone, triacetoneamine, cyclopentanone, cyclohexanone, dimethylcyclohexanone (giving rise, after aromatization, to 2,6-dimethylaniline), hydroxybutanone; (iii) aminoaldehydes, such as diisopropylaminoacetaldehyde; (iv) aromatic aldehydes and ketones, such as benzaldehyde, acetophenone, 4-methyl-acetophenone, indanone, dimethylindanone, orthophthalodialdehyde and meta-xylylenedialdehyde; (v) biobased aldehydes and ketones, such as 2-octanone, furfural, aldehydes derived from isosorbide and isomannide, or sugars themselves, such as glucose, lactose, mannose, saccharose and maltose.

The inventive amine is characterized by a low molar share of deuterium in the $R_1R_2HC-NH_2$ group or groups formed in step (c), which is $\leq$ 100 ppm, preferably in the range of from 10 to 95 ppm, more preferably in the range of from 15 to 90 ppm, most preferably in the range of from 20 to 80 ppm, especially 30 to 75 ppm, based on the total hydrogen content of said $R_1R_2HC-NH_2$ group or groups. Said amines are prepared by the process of the present invention comprising steps (a) to (c).

Said low deuterium molar share is introduced by step (a) of the process according to the present invention. Therefore, also the ammonia, prepared by reacting the hydrogen from step (a) with nitrogen in step (b) is characterized by this low deuterium molar share.

With the present invention environmentally friendly amines and an environmentally friendly process for making the same, wherein said process uses as little fossil energy as possible, are provided.

As mentioned above, it is important that the origin of the hydrogen and down-stream compounds obtained by clean energy can be tracked in a reliable way.

Today, the majority of hydrogen is produced from fossil fuels by steam reforming of natural gas and other light hydrocarbons, partial oxidation of heavier hydrocarbons, and coal gasification. According to the invention, hydrogen obtained by electrolysis is obtained by using non-fossil energy sources. It is expected that the electrification (power generation) of fossil sources will be fully replaced by the generation of power by non-fossil resources in the near future. The downstream products based on hydrogen, preferably amines and ammonia based on hydrogen obtained by electrolysis and hydrogen itself, can be distinguished by its deuterium molar share from amines, ammonia and hydrogen prepared by processes based on fossil energy, i.e. made by petrochemical processes.

The present invention therefore relates to the use of the molar share of deuterium in downstream compounds based on hydrogen for tracing the origin, especially the energetic origin, of the hydrogen bound in the downstream compounds based on hydrogen, wherein the downstream compounds are amines.

The present invention further relates to a process for tracing the origin, especially the energetic origin, of hydrogen bound in downstream compounds based on hydrogen by determining the molar share of deuterium in said downstream compounds based on hydrogen, wherein the downstream compounds are amines.

Tracing is in the meaning of the present invention synonymous with tracking.

The origin is in the meaning of the present invention the preparation method of the hydrogen employed, especially electrolysis and/or the energetic origin, i.e. non-fossil energy sources. As mentioned above, it is expected that the electrification (power generation) of fossil sources will be fully replaced by the generation of power by non-fossil resources in the near future. Hydrogen made by electrolysis is in this case hydrogen of non-fossil origin. Examples for non-fossil power sources are mentioned above.

The inventive process for tracing the origin, especially the energetic origin, of hydrogen and downstream compounds mentioned above may be employed as a single tracing (tracking) method or in combination with further tracing (tracking) methods.

The inventive processes of this application and the thus synthesized chemical compounds can be used in the synthesis of surfactants: Carbonyl compounds can be transformed into amines by reaction with ammonia, primary or secondary amines in the presence of the inventive hydrogen.

Transforming carbonyl compounds into amines using the inventive hydrogen can be carried out by the methods described in WO2023187051, WO2023187044, WO2009080512 and the references cited therein. The carbonyl compounds to be transformed into amines using the inventive hydrogen can, for example, be selected from linear or branched chain aldehydes, preferably C8 to C26 aldehydes, sugar derivatives, preferably derivatives of mono- or disaccharides, linear or branched chain C3 to C26 keto-compounds. Amines to be used in the reductive amination process can for example be selected from ammonia, primary or secondary alkyl amines, preferably linear or branched chain primary amines and linear or branched chain secondary amines, proteinogenic amino acids such as alanine, arginine, aspartic acid, asparagine, cysteine, selenocysteine, glycine, glutamic acid, glutamine, histidine, methionine, leucine, isoleucine, lysine, phenylalanine, proline, serine, threonine, tryptophan, tyrosine and valine, in particular proline; and non-proteinogenic amino acids, amino acid derivatives and amino acid analogues, such as glyphosate, hydroxyproline, pipecolic acid, pyrrolysine, ornithine, carnitine, p-N-methylamino-alanine, N-methyl-alanine, sarcosine, taurine and N-methyl taurine, in particular N-methyl taurine, N-methyl-alanine and sarcosine.

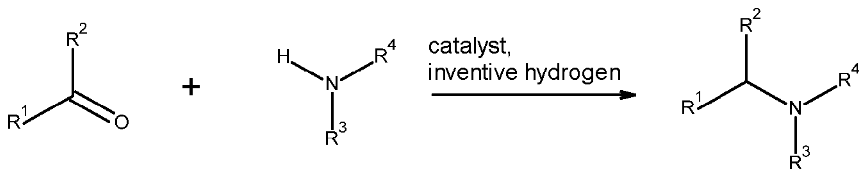


$R^1$ is an organic moiety, $R^2$ to $R^4$ can be organic moieties or hydrogen

Examples for using the inventive hydrogen in reaction of amines and carbonyl compounds to make amines that can be used to manufacture surface active compounds are given in the table below.

| Carbonyl compound | Amine compound | Conditions |
|---|---|---|
| Dodecanal | L-Proline | Pd/C, THF, 5 bar $H_2$, 30 °C 12h |
| Nonanal | Sarcosine | Pd/C, THF, 20 bar $H_2$, 30 °C, 24h |
| Nonanal | N-Methyl taurine | Pd/C, THF, 20 bar $H_2$, 50 °C, 24h |
| Dodecanal | rac. pipecolic acid | Pd/C, THF, 5 bar $H_2$, 30 °C, 24h, |

Such Amines can have surface active properties by themselves, or they can be used to produce surface active compounds, for example surfactants, for example by converting the amino group to the corresponding amine oxide or by forming amides with carboxylates or by alkoxylation.

Furthermore, the products according to the process described herein can be converted to a product as described on page 18, line 29 to page 26, line 29 of European patent application No. EP24164893.0.

Suitable deuterium molar shares of the amines are mentioned in the present application.

Examples

I Hydrogen Production

Experimental Setup and Method: Electrolysis cell design and Hydrogen production conditions

1) Polymer Electrolyte Membrane / Proton Exchange Membrane Electrolysis (PEM)

Electrolysis Cell:_Water electrolysis was conducted with a circular commercial PEM electrolysis cell (model ZE 200, Sylatech Analysetechnik GmbH, 0.007 $m^2$ active area). The cell stack was sealed with O-rings and wrapped with heat insulation fabric for isothermal operating conditions. A Nafion® 117 standard membrane (supplier DuPont, dry thickness 180 microns) was assembled by HIAT GmbH with catalytic active coating materials iridium (19 g/$m^2$) and platinum (8 g/$m^2$). Water distribution at the anode half-cell was realized with a titanium mesh. Before the polymer electrolyte membrane, a porous transport layer with sintered titanium fiber material is used for controlled flow while a porous graphite plate was situated on the cathode-side.

Experimental Conditions: Water with controlled temperature was supplied at constant flow of 9.5 g/h to the anode compartment. The cell pressure on cathode and anode side was controlled with PC valves. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic cell-water was re-cycled, whereas on the cathode the separated water was drained. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

2) Alkaline Electrolysis Cell (AEC)

Electrolysis Cell:_Alkaline water electrolysis was conducted with a circular AEC electrolysis cell (model Electro MP Cell, supplier ElectroCell Europe A/S, 0.01 $m^2$ electrode area). As electrodes Nickel 2.4068 material was used and separated by a commercial standard Zirfon Perl UTP 500 membrane (open mesh polyphenylene sulfide fabric symmetrically coated with a mixture polymer / zirconium oxide; thickness 500 microns; 0.023 $m^2$ active area; supplier Agfa-Gevaert N.V.) in zero-gap cell configuration.

Experimental Conditions: Alkaline water (32 wt% potassium hydroxide, technical standard grade) with controlled temperature was supplied at constant flow of 27.8 kg/h to the anode and cathode compartment. The cell pressure on cathode and anode side was equalized via PC valve control. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic and cathodic cell-water was re-cycled. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

3) Anion Exchange Membrane / Alkaline Electrolyte Membrane Electrolysis (AEM)

Electrolysis Cell: The AEM experiments were executed in a commercial, fully automated 2.4 kW EL 4.0 cell supplied by Enapter GmbH, 10117 Berlin and a 1 wt% potassium hydroxide (standard grade) electrolyte solution.

Test station:_As recommended by the supplier the EL 4.0 electrolyzer is run with a 1 wt% potassium hydroxide (technical standard grade) solution, hydrogen production at operating conditions (experimental conditions such as temperature and pressure settings see table) was 480 l/h at approx. 400 ml water consumption. The evolved hydrogen gas was treated with desiccant dryers, ensuring complete separation of moisture in the electrolysis gas flow to yield < 0,03 wt% water in the hydrogen gas stream. Small gas samples of the dehydrated cathodic hydrogen gas were taken from the continuous flow with an auto-sampler at regular intervals to conduct hydrogen gas analytics described below.

4) Solid Oxide Electrolysis Cell (SOE)

Solid Oxide cell stacks from Elcogen- Elco Stack (Elcogen OY, Vantaa 01510 Finland), were used and a E3000 unit was operated in reverse mode at 700°C and 35A electrolyzing current; Anode functional composition due to the supplier is NiO/YSZ. Cathode is of LSC type [La(Sr)CoO3]: The principle is also described in Novel high-performance solid oxide fuel cells with bulk ionic conductance dominated thin-film electrolytes - ScienceDirect (www.sciencedirect.com/science/article/pii/S037877531201097X); D. Stover et al, Journal of Power Sources; Volume 218, 15 November 2012, Pages 157-162.

The hydrogen stream was used with no further purification/dryer. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

The results of the hydrogen production are shown in Table 1.

Table 1

| Electrolyser type | Example I) PEM electrolyser | Example II) PEM electrolyser | Example III) Alkaline electrolyser | Example IV) Alkaline electrolyser | Example V) AEM | Example VI) AEM | Example VII) Solid oxide |
|---|---|---|---|---|---|---|---|
| Water-type/deuterium content | VSMOW/155,76 ppm D | Selected* surface water/139 ppm D | VSMOV/155,76 ppm D | Selected* surface water/139ppm D | VSMOW/155,76 ppm D | Selected* surface water/139 ppm D | Selected* surface water/139 ppm D |
| Current density [A/cm2] | 1.35 | 1.35 | 0.8 | 0.8 | 1.6 | 1.6 | 0.78 |
| Applied voltage [V] | 1.9 | 1.9 | 2.15 | 2.15 | 1.9 | 1.9 | 1.35 |
| Operating temperature [°C] | 68 | 68 | 81 | 81 | 45 | 45 | 710 |
| Cell pressure [bar] | 23 | 23 | 25 | 25 | 35 | 35 | 1.3 |
| Voltage efficiency [%] | 57 | 57 | 53 | 53 | 66 | 66 | 81 |
| Electrical efficiency [kWh/kg H2] | 52 | 52 | 48.5 | 48.6 | 69 | 69 | 47 |
| Deuterium content in hydrogen [ppm] | 86 | 77 | 95 | 87 | 74 | 66 | 38 |

* Isar river surface water (Munich) collected in winter season January 2021.

Experimental Setup and Method: "D content (Deuterium content) in samples"

The following method descriptions apply for determination of the molar share of deuterium based on the total hydrogen content (Deuterium content) of gas and liquid samples. The isotopic H/D-share analysis is based on mass spectroscopy. Two different methods are used: method A for gas samples and method B for liquid samples.

For determination of the "D content in gas and liquid samples" it is of crucial importance not to contaminate the samples e.g. with ambient humidity or other ambient components containing hydrogen or deuterium. Therefore gas-tight materials and sealings must be used with clean sample containers to avoid any cross-contamination. Therefore, before filling and sealing a sample container it must be flushed at least 20 times the sample container volume with the gas or liquid stream to be analyzed. The same is valid for the experimental setup of the gas sampler and mass spectrometer. Utmost care must be taken to avoid cross-contamination e.g. via condensation of humidity. The analytical setup from sampling to mass spectrometry is validated with known reference samples.

Method A) Gas samples

Total Deuterium from HD and $D_2$ in hydrogen gas samples was determined via ultra-high resolution quadrupole mass spectrometry using a Hiden DLS-20 (Hiden Analytical Ltd., Warrington, Cheshire, UK) analyzer setup. The general method setup is described in C.C. Klepper, T.M. Biewer, U. Kruezi, S. Vartanian, D. Douai, D.L. Hillis, C. Marcus, Extending helium partial pressure measurement technology to JET DTE2 and ITER; Rev. Sci. Instrum., 87 (11) (2016); doi: 10.1063/1.4963713. For the hydrogen gas samples the threshold ionization mass spectrometry mode (TIMS) was used as described in S. Davies, J.A. Rees, D.L. Seymour; Threshold ionization mass spectrometry (TIMS); A complementary quantitative technique to conventional mass resolved mass spectrometry; Vacuum, 101 (2014), pp. 416-422; doi: 10.1016/j.vacuum.2013.06.004. Sensitivity is +/-1 ppm.

Method B) Liquid samples

Analysis of liquid samples (ammonia, amines and carbonyl compounds) was executed via isotope ratio monitoring gas chromatography/mass spectrometry (IRMS). Therefore a DELTA V PLUS CF-IRMS mass spectrometer was used. This mass spectrometer with magnetic sector with continuous flux DELTA V PLUS CF-IRMS is used to measure the isotopic ratio of D/H.

Measurement of D/H in a continuous He-flow mode needs the complete removal of low energy 4 He+ ions from the HD+ ion beam at m/z 3). The method is described in RAPID COMMUNICATIONS IN MASS SPECTROMETRY Rapid Commun. Mass Spectrom. 13, 1226-1230 (1999), W.A. Brandt et al. Sensitivity is within +/-3 ppm.

Method C) Analysis of isotope ratios in starting materials and products

Analysis of isotope ratios in starting materials and products was furthermore executed via SNIF-NMR related methods ("site-specific natural isotope fractionation studied by nuclear magnetic resonance") using high resolution $^{2}$H-NMR (Bruker Avance NEO 600 MHz NMR Spectrometer and Bruker Avance III HD 700 MHz NMR Spectrometer both equipped with TCI probes).

Principles of this technology are described in Gérard J. Martin, Serge Akoka, and Maryvonne L. Martin; SNIF-NMR Part 1: Principles; in Graham A. Webb (ed.), Modern Magnetic Resonance, Springer (2008) pp 1651-1658 as well as Maryvonne Martin, Benli Zhang, and Gérard J. Martin; SNIF-NMR Part 2: Isotope Ratios as Tracers of Chemical and Biochemical Mechanistic Pathways; in Graham A. Webb (ed.), Modern Magnetic Resonance, Springer (2008) pp 1659-1667 and Gérard J. Martin, Maryvonne L. Martin and Gérald Remaud; SNIF-NMR Part 3: From Mechanistic Affiliation to Origin Inference; in Graham A. Webb (ed.), Modern Magnetic Resonance, Springer (2008) pp 1669-1680.

II Production of Amines

Amines are prepared with catalytic synthesis processes in industrial scale as described above.

By using non-fossil-based energy for electrolytic hydrogen in both the ammonia and the hydrogen synthesis for the reductive amination of aldehydes and ketones, a substantial fraction of the hydrogen is originating from the electrolytic hydrogen with a low deuterium content. Especially for ammonia this is a big difference since the fossil-based production process relies on synthesis gas made from fossil natural gas and additionally large quantities of steam. This steam reforming step is not required for the non-fossil ammonia route based on pure hydrogen and nitrogen.

In all of the large-scale commercial production processes in question using non-fossil hydrogen no significant additional amounts of hydrogen-species are introduced in the "input/output" process material balance. This is required in order to minimize output of undesired wastewater or other liquid and gaseous emission streams and to ensure high yields

with purity according to the product specification.

In case water is used as processing aid for stripping/scrubbing/cleaning or quenching/condensing of process-internal streams in e.g. the ammonia or amines synthesis steps this water is always run in an almost completely closed cycle with minimum purge ensuring insignificant cross-contamination with deuterium.

## Examples

Nl denotes volumes in litres at standard conditions

### Example 1: Mono-Isopropylamin (MIPA)

To carry out the tests continuously, a high-pressure reactor with an internal diameter of 45 mm and a length of 350 cm was used. A thermoelement with a diameter of 12 mm was attached in the center and along the axis of the reactor. The reactor was packed in the lower part with 700 ml of stainless steel Pall rings, on top of this 3500 ml=3509 g of catalyst (76% by weight of Al2O3, 4% by weight of copper oxide, calculated as CuO, 10% by weight of CoO and 10% by weight of NiO) as extrudates were introduced, and a further 700 ml of stainless steel Pall rings were introduced as top layer. Acetone, ammonia and hydrogen were fed into the reactor from top to bottom. The reactor pressure was adjusted by supplying hydrogen. Downstream of the reactor, the reaction mixture was cooled, decompressed to atmospheric pressure and analyzed.

The detailed test conditions and the results are to be found in the following table:

| T No | p bar | T °C | SV Kg/(1*h) | MR $NH_3$/Ac. | MIPA % (*) | DIPA % (*) | Acetone % (*) | iPrOH % (*) | Others % (*) |
|---|---|---|---|---|---|---|---|---|---|
| 1 | 200 | 150 | 0.1 | 7.8 | 98.9 | 0.05 | 0.01 | 0.9 | 0.1 |
| 2 | 200 | 130 | 0.35 | 2.1 | 96.8 | 0.7 | 0 | 2.2 | 0.3 |
| 3 | 65 | 135 | 0.1 | 4.6 | 97.5 | 0.05 | 0 | 2.1 | 0.3 |
| 4 | 50 | 130 | 0.1 | 6.0 | 97.0 | 0.2 | 0 | 2.2 | 0.6 |
| 5 | 50 | 130 | 0.2 | 3.0 | 97.4 | 0.1 | 0 | 1.7 | 0.8 |
| 6 | 50 | 150 | 0.35 | 2.1 | 97.5 | 0.1 | 0.2 | 1.2 | 0.8 |
| 7 | 50 | 160 | 0.35 | 2.1 | 97.3 | 0.2 | 0.02 | 1.8 | 0.6 |
| 8 | 40 | 135 | 0.1 | 4.6 | 97.5 | 0.05 | 0 | 2.1 | 0.3 |
| 9 | 40 | 140 | 0.2 | 4.6 | 98.1 | 0.02 | 0 | 1.6 | 0.3 |
| 10 | 40 | 140 | 0.4 | 4.6 | 97.8 | 0.02 | 0.3 | 1.3 | 0.4 |
| 11 | 35 | 140 | 0.35 | 2.1 | 97.1 | 0.15 | 0.15 | 1.5 | 1.0 |
| 12 | 35 | 120 | 0.35 | 2.1 | 96.8 | 0.15 | 0.5 | 1.2 | 1.0 |

SV = space velocity in kg of acetone per l of catalyst (bulk volume) and per hour
MR = molar ratio
Ac. = acetone
DIPA = diisopropylamine
iPrOH = isopropanol
Others = other byproducts
(*) GC column: 30 m Stabilwax DB Amine, 0.25 µta internal diameter, 0.5 µta film thickness

### Example 2: Isophorone diamine from Isophorone

The process for preparing isophorone diamine (= 3-aminomethyl-3,5,5-trimethyl-cyclohexylamine) from isophorone nitrile (3-cyano-3,5,5-trimethyl-cyclohexanone) is carried out in two separate reaction spaces. In the first reaction space, 3-cyano-3,5,5-trimethyl-cyclohexanone is reacted with excess ammonia in the presence of an acidic metal oxide catalyst at a temperature of from 20 to 150°C and at a pressure of from 15 bis 500 bar. The obtained reaction products are hydrogenated with hydrogen in the presence of excess ammonia on a cobalt, nickel, ruthenium or other precious metal containing catalyst, optionally with basic components or on a neutral or basic carrier, at temperatures of from 60 to 150 °C

and a pressure of from 60 to 300 bar.

Example 3: Isophorone diamine from Isophorone

A vertical tube reactor (diameter: 16 mm; filling height: 50 cm; oil-heated double jacket) was filled with 176.7 g (100 ml) of a basic cobalt full-contact (CoO with 5 % $Mn_2O_3$ and 1.4 % $Na_2O$) in the form of 1 to 1,5 mm split. For the reduction of the catalyst, the temperature was raised stepwise within 23 h from 100 to 330 °C and held for 30 h at 330°C while flowing 150 Nl/h hydrogen through the reactor at 100 bar. Through a tube reactor (diameter: 16 mm, filling height: 50 cm, oil-heated double jacket) filled with 70.0 g (100 ml) of alumina in in the form of 1,5 mm-strands and mounted upstream from the hydrogenation reactor, 13.8 g molten isophorone nitrile (purity 99.0 %) and 303.0 g liquid ammonia were hourly pumped at 250 bar and at a temperature of 80°C from bottom to top. Then 60 Nl/h (2.7 mol) of hydrogen were fed together with the output from the upstream-mounted imination reactor at a pressure of 250 bar and a temperature of 130 °C from bottom to top through the hydrogenation reactor. After depressurizing to normal pressure, ammonia was distilled off. The product mixture of the hydrogenation contained, according to GC analysis, 97.7 % isophorone diamine und 0.3 % 1,3,3-trimethyl-6-azabicyclo[3.2.1 ]octane, corresponding to a diamine-yield of 98.7%.

Example 4: Isophorone diamine from Isophorone

A vertical tube reactor (diameter: 16 mm, filling height: 100 cm, oil-heated double jacket) was filled with 354 g (200 ml) of a basic cobalt full-contact (CoO with 5 % $Mn_2O_3$ and 1.4 % $Na_2O$) in the form of 1 to 1.5 mm split. For the reduction of the catalyst, the temperature war raised stepwise within 23 h from 100 to 330 °C and held for 30 h at 330°C while flowing 150 Nl/h hydrogen through the reactor at 100 bar. Through a tube reactor (diameter: 16 mm; filling height: 20 cm; oil-heated double jacket) filled with 25.4 g (40 ml) of $TiO_2$ (Anatas) in in the form of 1,5 mm-strands and mounted upstream of the hydrogenation reactor, 40 g molten isophorone nitrile (purity 99.0 %) and 102 g liquid ammonia were hourly pumped at 250 bar and at a temperature of 110°C from bottom to top. Then 100 Nl/h (2.7 mol) of hydrogen were fed together with the output from the upstream-mounted imination reactor at a pressure of 250 bar and a temperature of 110 °C from bottom to top through the hydrogenation reactor. After depressurizing to normal pressure, ammonia was distilled off. The hydrogenation product mixture contained, according to GC analysis, 97.5 % isophorone diamine. The output was collected over 73 h and separated by rectification in a 30 cm packed fractionating column (3 mm glass rings). 2864 g of isophorone diamine were obtained, corresponding to a yield of 96.2 % of the theory.

Example 5: 4-Amino-2,2,6,6-tetramethylpiperidine (TAD) from 2,2,6,6-tetramethyl-4-piperidone (TAA)

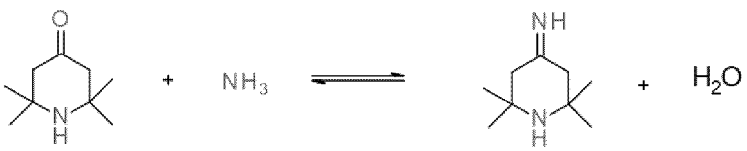

TAA Imin

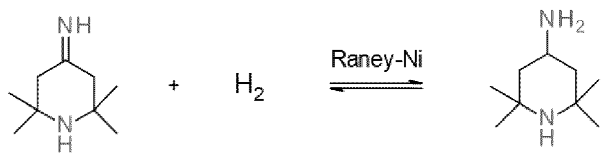


Imin TAD

An aqueous suspension of catalyst (Raney-Nickel) was fed into the reactor. In another vessel an aqueous ammonia solution was prepared by dissolving ammonia in water and added afterwards to the suspension of the catalyst. Next, this reactor was pressurized with hydrogen and heated to approximately 80°C.

Afterwards 2,2,6,6-tetramethyl-4-piperidone (TAA) as a melt dosing into the reactor started and pressurization of the reactor with hydrogen continued. After the hydrogen uptake was completed, the reaction was allowed to continue for approximately 1 hour. Then, the reaction mixture was cooled to 50°C. For isolation of the 4-Amino-2,2,6,6-tetramethylpiperidine (TAD) the catalyst was removed by filtration. For further purification the raw product was first distillated under normal pressure to remove water and ammonia. Afterwards heavy boilers are removed by distillation under reduced

pressure (50 mbar).

Example 6: N,N'-Bis(2,2,6,6-tetramethyl-4-piperidinyl)-1,6-hexanediamine (T5) from 2,2,6,6-tetramethyl-4-piperidone (TAA) and 1,6-hexamethylendiamine (HMDA)

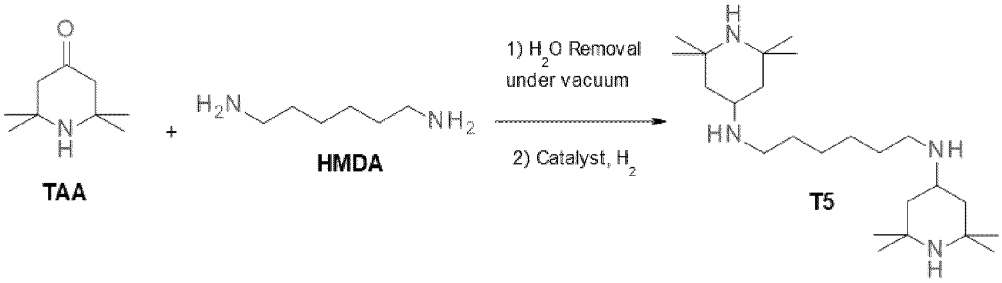


TAA HMDA T5

2,2,6,6- Tetramethyl-4-piperidone (TAA) was mixed with 1,6-hexamethylendiamine (HMDA) at a temperature from 20 to 100°C and the water of reaction (amount between 50-90%) was distilled off with vacuum. The ketimine solution was then hydrogenated with Pt or Pd on charcoal at 65 - 150°C, pressure from 5 to 50 bar maximum pressure. After cooling the product was filtered from the catalyst.

Example 7: 4-(Butylamino)-2,2,6,6-tetramethylpiperidine (T7) from 2,2,6,6-tetramethyl-4-piperidone (TAA) and butylamine (BUA)

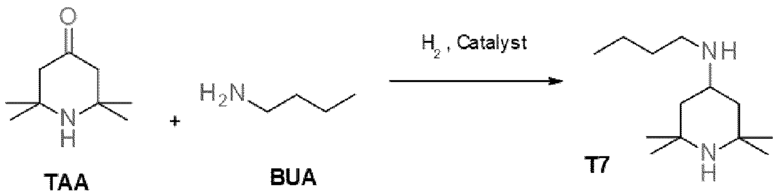


TAA BUA T7

2,2,6,6-Tetramethyl-4-piperidone (TAA) was mixed with butylamine (BUA) at temperature from 20 to 60°C to form the corresponding Schiff Base which was hydrogenated in the presence of Pt or Pd catalyst supported on charcoal at 65 -150 °C, under pressure from 5 to 50 bar maximum. After cooling the product was filtered from the catalyst.

## Claims

**1.** A process for the preparation of amines comprising the following steps:

(a) providing hydrogen with a molar share of deuterium ≤ 100 ppm, based on the total hydrogen content, by electrolysis of water using electrical power generated at least in part from non-fossil energy,
(b) reacting the hydrogen from step (a) with nitrogen to form ammonia,
(c) reacting the ammonia from step (b) with a carbonyl compound (I)

$$R_1R_2C=O \quad (I)$$

in the presence of hydrogen from step (a) to form the corresponding amine (II)

$$R_1R_2HC\text{-}NH_2 \quad (II).$$

**2.** The process of claim 1, wherein the electrical power is generated from wind power, solar energy, biomass, hydropower

and geothermal energy.

**3.** The process according to claim 1 or 2, wherein hydrogen is provided by polymer electrolyte membrane water electrolysis.

**4.** The process according to claim 3, wherein hydrogen is provided by proton exchange membrane water electrolysis (PEMWE) or anion exchange membrane water electrolysis (AEMWE).

**5.** The process according to any one of claims 1 to 4, wherein the molar share of deuterium in the hydrogen provided in step (a) is in the range of from 10 to 95 ppm, preferably in the range of from 10 to 90 ppm, more preferably in the range of from 20 to 80 ppm, and most preferably in the range of from 30 to 75 ppm, based on the total hydrogen content.

**6.** The process according to any one of claims 1 to 5, wherein step (c) is carried out as heterogeneously or homogeneously catalyzed reaction.

**7.** The process according to any one of claims 1 to 6, wherein the carbonyl compound (I) is selected from

(i) aliphatic aldehydes, preferably acetaldehyde, propionic aldehyde, butyraldehyde, isobutyraldehyde, hexanal, 2-ethylhexanal, formylpinane, or dialdehydes like glutaraldehyde
(ii) aliphatic ketones, preferably acetone, isophorone nitrile, adamantone, menthone, triacetoneamine, cyclopentanone, cyclohexanone, dimethylcyclohexanone, hydroxybutanone;
(iii) aminoaldehydes, preferably diisopropylaminoacetaldehyde;
(iv) aromatic aldehydes and ketones, preferably benzaldehyde, acetophenone, 4-methyl-acetophenone, indanone, dimethylindanone, orthophthalodialdehyde and meta-xylylenedialdehyde;
(v) biobased aldehydes and ketones, preferably 2-octanone, furfural, aldehydes derived from isosorbide and isomannide, and sugars, such as glucose, lactose, mannose, saccharose and maltose.

**8.** Amines, obtainable in the process according to any one of claims 1 to 7.

**9.** Amines according to claim 8, wherein the molar share of deuterium in the $R_1R_2CH\text{-}NH_2$ group or groups formed in step (c) of the process according to any one of claims 1 to 7 is $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content of said $R_1R_2CH\text{-}NH_2$ group or groups.

**10.** Amines according to claim 8 or 9 selected from the group consisting of

a) alkylamines and aliphatic diamines, preferably ethylamine, diethylamine, propylamine, dipropylamine, butylamine, isobutylamine, dibutylamine, diisobutylamine, 2-ethylhexylamine, bis(2-ethylhexyl)amine, tris(2-ethylhexyl)amine, octylamine, 2-propylheptylamine, isononylamine, 3,5,5-trimethylhexylamine, tridecyclamine, ditridecylamine, diethylaminoethylamine, diisopropylaminoethylamine, isopropylamine, diisopropylamine, adamantylamine, tridecylamine, cyclopentylamine, cyclohexylamine, menthylamine, aminated glucose, piperidine, triacetonediamine, isophoronediamine and aminomethylpinane;
b) alkanolamines, preferably 2-aminobutanol, 3-aminobutanol, 2-aminopropanol and 3-aminopropanol;
c) aromatic amines, preferably benzylamine, 1-phenylethylamine, aminoindane, dimethylaminoindane, benzylamine, 4-methyl-2-phenylethylamine, 2,6-dimethylaniline, orthophthalodiamine and meta-xylylene diamine;
d) biobased amines, preferably 2-octylamine, furfurylamine, tetrahydrofurfurylamine, isosorbide diamine, isomannide diamine, aminated sorbitol, bisaminomethylfurane and bisaminomethyltetrahydrofurane.

**11.** The use of the molar share of deuterium in downstream compounds based on hydrogen for tracing the origin, especially the energetic origin, of hydrogen bound in the downstream compounds based on hydrogen, wherein the downstream compounds are amines.

**12.** The use according to claim 11, wherein the downstream compounds are amines according to any one of claims 8 to 10.

**13.** A process for tracing the origin, especially the energetic origin, of hydrogen bound in downstream compounds based on hydrogen by determining the molar share of deuterium in said downstream compounds based on hydrogen, wherein the downstream compounds are amines.

**14.** The process according to claim 13, wherein the downstream compounds are amines according to any one of claims 8 to 10.

Europäisches Patentamt
European Patent Office
Office européen des brevets

# EUROPEAN SEARCH REPORT

Application Number
EP 24 20 7972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2011/136097 A1 (SMAJLOVIC IVAN [RS]) 9 June 2011 (2011-06-09)<br>* the whole document * | 11-14 | INV.<br>C25B1/04<br>C25B15/08<br>C01B4/00<br>C01C1/04<br>C07B59/00<br>C07C209/26 |
| A | FELLOWS S K ET AL: "Availability of large quantities of low-deuterium hydrogen, and possible uses", INTERNATIONAL JOURNAL OF HYDROGEN ENERGY, ELSEVIER, AMSTERDAM, NL, vol. 6, no. 1, 1 January 1981 (1981-01-01), pages 67-71, XP025591252, ISSN: 0360-3199, DOI: 10.1016/0360-3199(81)90098-7 [retrieved on 1981-01-01]<br>* the whole document * | 11-14 | |
| Y | CN 112 939 784 B (SHANXI TIEFENG CHEMICAL CO LTD) 2 December 2022 (2022-12-02)<br>* the whole document * | 1-14 | |
| Y | US 2011/178131 A1 (JOHNSON DALE JAMES [GB]) 21 July 2011 (2011-07-21)<br>* the whole document * | 1-14 | **TECHNICAL FIELDS SEARCHED (IPC)**<br>C25B<br>C07B<br>C01C<br>C01B<br>C07C |
| Y | US 2020/148547 A1 (PAPILE CHRISTOPHER [US]) 14 May 2020 (2020-05-14)<br>* the whole document * | 1-14 | |
| A,P | WO 2023/213713 A1 (BASF SE [DE]) 9 November 2023 (2023-11-09)<br>* the whole document * | 11-14 | |

The present search report has been drawn up for all claims

1

| Place of search | Date of completion of the search | Examiner |
|---|---|---|
| Munich | 5 March 2025 | Ritter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 20 7972

This annex lists the patent family members relating to the patent documents cited in the above-mentioned European search report. The members are as contained in the European Patent Office EDP file on
The European Patent Office is in no way liable for these particulars which are merely given for the purpose of information.

05-03-2025

| Patent document cited in search report | Publication date | Patent family member(s) | Publication date |
|---|---|---|---|
| US 2011136097 A1 | 09-06-2011 | NONE | |
| CN 112939784 B | 02-12-2022 | NONE | |
| US 2011178131 A1 | 21-07-2011 | EP 2344483 A1 | 20-07-2011 |
| | | JP 2012504574 A | 23-02-2012 |
| | | US 2011178131 A1 | 21-07-2011 |
| | | WO 2010037723 A1 | 08-04-2010 |
| US 2020148547 A1 | 14-05-2020 | NONE | |
| WO 2023213713 A1 | 09-11-2023 | AU 2023266064 A1 | 14-11-2024 |
| | | CN 119156370 A | 17-12-2024 |
| | | EP 4519241 A1 | 12-03-2025 |
| | | KR 20250006960 A | 13-01-2025 |
| | | WO 2023213713 A1 | 09-11-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0713861 A1
- EP 3050870 A1
- EP 0449089 A1
- US 20010003137 A1
- EP 1312600 A
- EP 1312599 A
- US 20120245390 A1
- WO 2016189129 A1
- US 20040267051 A
- US 3275554 A
- DE 2125039 A
- DE 3611230 A
- WO 2023187051 A
- WO 2023187044 A
- WO 2009080512 A
- EP 24164893


### Non-patent literature cited in the description


- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. HARADA et al.** *International Journal of Hydrogen Energy*, 2020, vol. 45, 31389-31 395
- **H. SATO et al.** *International Journal of Hydrogen Energy*, 2021, vol. 46, 33 689-33 695
- **ROOSE, P.** ; **ELLER, K.** ; **HENKES, E.** ; **ROSSBACHER, R.** ; **HOKE, H.** *Amines, Aliphatic, in Ullmann's Encyclopedia of Industrial Chemistry*, 2015, https://doi.org/10.1002/14356007.a02_001.pub2
- **ROOSE, P.** ; **TURCOTTE, M.G.** Amines, lower aliphatic, in Kirk-Othmer Encyclopedia of Chemical Technology. John Wiley & Sons, Inc, 2016
- **R. PATIL** ; **S. ADIMURTHY**. *Asian J. Org. Chem.*, 2013, vol. 2, 726-744
- Fixed-Bed Reactors. Ullmann's Encyclopedia of Industrial Chemistry, vol. B 4, 199-238
- *Chem. Eur. J.*, 2014, vol. 20, 245-252
- **D. STOVER et al.** *Journal of Power Sources*, 15 November 2012, vol. 218, 157-162
- **C.C. KLEPPER** ; **T.M. BIEWER** ; **U. KRUEZI** ; **S. VARTANIAN** ; **D. DOUAI** ; **D.L. HILLIS** ; **C. MARCUS**. Extending helium partial pressure measurement technology to JET DTE2 and ITER. *Rev. Sci. Instrum.*, 2016, vol. 87 (11)
- **S. DAVIES** ; **J.A. REES** ; **D.L. SEYMOUR**. Threshold ionization mass spectrometry (TIMS); A complementary quantitative technique to conventional mass resolved mass spectrometry. *Vacuum*, 2014, vol. 101, 416-422
- **W.A. BRANDT**. RAPID COMMUNICATIONS IN MASS SPECTROMETRY. *Rapid Commun. Mass Spectrom*, 1999, vol. 13, 1226-1230
- SNIF-NMR Part 1: Principles. **GÉRARD J. MARTIN** ; **SERGE AKOKA** ; **MARYVONNE L. MARTIN**. Modern Magnetic Resonance. Springer, 2008, 1651-1658
- SNIF-NMR Part 2: Isotope Ratios as Tracers of Chemical and Biochemical Mechanistic Pathways. **MARYVONNE MARTIN** ; **BENLI ZHANG** ; **GÉRARD J. MARTIN**. Modern Magnetic Resonance. Springer, 2008, 1659-1667
- SNIF-NMR Part 3: From Mechanistic Affiliation to Origin Inference. **GÉRARD J. MARTIN** ; **MARYVONNE L. MARTIN** ; **GÉRALD REMAUD**. Modern Magnetic Resonance. Springer, 2008, 1669-1680